# EUROPÄISCHE PATENTSCHRIFT

(11) **EP 2 499 119 B1**
(45) Veröffentlichungstag und Bekanntmachung des Hinweises auf die Patenterteilung: **06.01.2016**
(21) Anmeldenummer: 10781587.0
(22) Anmeldetag: 24.09.2010
(51) Int. Cl.: C07D 251/60

(54) **VERFAHREN ZUR KONTINUIERLICHEN HERSTELLUNG VON MELAMIN**
METHOD FOR THE CONTINUOUS PRODUCTION OF MELAMINE
PROCÉDÉ DE PRODUCTION CONTINUE DE MÉLAMINE

(30) Priorität: 10.11.2009 DE 102009052420
(43) Veröffentlichungstag der Anmeldung: 19.09.2012
(73) Patentinhaber: Air Liquide Global E&C Solutions Germany GmbH, 60439 Frankfurt am Main (DE)
(72) Erfinder: CASTILLO-WELTER, Frank, 61381 Friedrichsdorf (DE); STEDEN, Christoph, 61440 Oberursel (DE); WALTER, Dominic, 64289 Darmstadt (DE); EHRING, Georg, 60316 Frankfurt (DE); MÜLLER-HASKY, Martin, 63150 Heusenstamm (DE)
(86) Internationale Anmeldenummer: PCT/DE2010/001135
(87) Internationale Veröffentlichungsnummer: WO 2011/057596

(56) Entgegenhaltungen:
- WO-A1-2006/119815
- WO-A1-2008/098732

## Beschreibung

Die Erfindung betrifft ein Verfahren zur kontinuierlichen Herstellung von Melamin, wobei der als Ausgangsmaterial verwendete, flüssige Harnstoff in einem Wirbelbettreaktor, in Anwesenheit eines Katalysators, unter Wärmezufuhr und unter Zugabe von Ammoniak in ein Prozessgas, bestehend hauptsächlich aus Melamin, Ammoniak, Kohlendioxid, dem Zwischenprodukt Isocyansäure und Nebenprodukten, umgewandelt wird und wobei dieses Prozessgas, nachdem aus ihm die Nebenprodukte und das Melamin abgetrennt wurden, in einen Wäscher geleitet wird, in dem es mit flüssigem Harnstoff gewaschen und vom Isocyansäuregehalt befreit wird, wobei das Prozessgas, nach Verlassen des Wäschers, zum Teil als Verwirbelungsgas im Wirbelbettreaktor eingesetzt wird, zum Teil als Kühlgas im Kristallisator eingesetzt wird und zum Teil aus dem Prozessgaskreislauf ausgeschleust wird.

Verfahren zur kontinuierlichen Herstellung von Melamin aus Harnstoff sind bekannt. Ein Beispiel dafür ist das sogenannte BASF-Niederdruckverfahren, wie es in Ullmann's Encyclopedia of Industrial Chemistry, Sixth Edition, Chap. 4.1.1., 1998 Electronic Release beschrieben ist.

Das Verfahren nach dem Stand der Technik läuft bei hohen Temperaturen in der Gasphase ab. Dabei wird das Ausgangsmaterial, der Harnstoff, flüssig in einen Wirbelbettreaktor eingegeben, darin von einem NH₃ - CO₂- Prozessgasgemisch durchwirbelt, bei Temperaturen von 390 bis 410°C ve rdampft, in Anwesenheit eines Aluminium-Katalysators, über das Zwischenprodukt Isocyansäure in Melamin umgewandelt, wobei als weitere Reaktionsprodukte Ammoniak und Kohlendioxid und als Nebenprodukte Melem und Melam entstehen. Dieses Gasgemisch wird, nach Verlassen des Wirbelbettreaktors, zunächst in einem Gaskühler auf ca. 340°C abgekühlt, um die Nebenprod ukte Melem und Melam auszukristallisieren, die in einem anschließend durchlaufenen Gasfilter, zusammen mit aus dem Wirbelbettreaktor mitgerissenen Katalysatorpartikeln, aus dem Gasstrom abgetrennt werden. Anschließend wird das Gas in einen Kristallisator geführt, in dem es zur Auskristallisierung des Melamins, auf 190 bis 220°C abgekühlt wird. Das Gemisch aus den restliche n gasförmigen Bestandteilen, Ammoniak, Kohlendioxid und Isocyansäure und auskristallisiertem, pulverförmigen Melamin wird aus dem Kristallisator in einen Abscheider geleitet, in dem das Melamin aus dem Gas abgetrennt und als Verfahrensprodukt ausgetragen wird. Die gasförmigen Bestandteile werden aus dem Abscheider mittels eines Gebläses in einen Wäscher geleitet, in dem das Gas mit flüssigem Harnstoff gewaschen wird, wobei die im Gas enthaltene Isocyansäure und sonstige Nebenprodukte der im Wirbelbettreaktor ablaufenden Reaktionen aus dem Gas herausgewaschen werden, in den flüssigen Harnstoff übergehen und damit im Prozess verbleiben. Aus dem Harnstoffkreislauf des Wäschers wird ein Teilstrom abgezweigt und, mit Ammoniak versetzt, als Ausgangstoff für die Melaminerzeugung in den Wirbelbettreaktor eingespeist. Das im Wäscher von Isocyansäureresten befreite Gasgemisch aus Ammoniak und Kohlendioxid wird zum Teil als Verwirbelungsgas im Wirbelbettreaktor verwendet und zum Teil als Kühlgas zum Auskristallisieren des Melamins in den Kristallisator eingespeist.

Im Wäscher wird das Prozessgas auf 135 bis 143°C he runtergekühlt, denn eine niedrige Temperatur, die möglichst knapp über der Schmelztemperatur des Harnstoffs von 130 - 135°C liegt, begünstigt eine weitgehende Umwandlung der Isocyansäure in Harnstoff. Außerdem ist eine möglichst niedrige Temperatur für den Einsatz als Kühlgas im Kristallisator vorteilhaft.

Kennzeichnend für dieses BASF-Niederdruckverfahren ist es, dass die gesamte Prozessgasmenge, nach dem Abscheiden des Melamins, über den, mit flüssigem Harnstoff betriebenen, Wäscher geführt wird, wobei die gasförmigen und festen Produkt- und Nebenproduktreste in den Harnstoff übergehen und, indem sie mit ihm in den Wirbelbettreaktor eingespeist werden, im Prozesskreislauf verbleiben und nicht mit dem Überschussgas aus dem Prozess ausgeschleust werden und verloren gehen. Die Produkt- und Nebenproduktreste werden im Wäscher, zum größten Teil, wieder in Harnstoff umgewandelt, sodass vermieden wird, dass diese Stoffe immer wieder den heissen Wirbelbettreaktor durchlaufen und dabei chemische Verbindungen bilden, die das Melamin verunreinigen könnten.

Nachteilig an dieser Behandlung des gesamten Prozessgases im Wäscher ist es, dass dadurch auch dem Förderorgan des Wirbelbettreaktors ein mit Harnstoff gesättigtes Prozessgas vorgelegt wird, sodass an dessen Eintritt immer wieder störende Harnstoffablagerungen gebildet werden.

Bei einem anderen Verfahren zur Herstellung von Melamin, das in der chinesischen Offenlegungsschrift CN 1188761A, Jiang Dazhou u.a., dargestellt ist, hat man diesen Nachteil, d.h. die Neigung zu Harnstoffablagerungen, vermieden, indem das als Verwirbelungsgas verwendete Prozessgas vollständig am Harnstoffwäscher vorbeigeführt wird. Bei diesem Verfahren wird das Prozessgas, nach Verlassen des Harnstoffwäschers, vollständig, als Kühlgas, durch den Melaminkristallisator geleitet, wird dabei aufgeheizt und wird mit der dabei erreichten Temperatur dem Förderorgan des Wirbelbettreaktors vorgelegt.

Nachteilig an diesem Verfahren ist, dass die Temperatur des Prozessgases dabei zwangsläufig der im Kristallisator herrschenden Gastemperatur entspricht und nicht, davon unabhängig, auf die für den Verdichter bzw. das Gebläse am besten geeignete Temperatur eingestellt werden kann.

Grundsätzlich ist die Begrenzung der Gastemperatur nach oben für die Herstellungskosten und für die Betriebssicherheit eines Verdichters oder Gebläses sehr wichtig. Eine Gastemperatur von etwa 200°C sollte nicht überschritten werden, da bei höheren Temperaturen die Anforderungen, und damit die Kosten, an das Förderorgan stark steigen.

Aufgabe der vorliegenden Erfindung ist es daher, das Verfahren dahingehend zu verbessern, dass die Temperatur des Gases am Eintritt des Verdichters oder des Gebläses des Wirbelschichtreaktors so eingestellt werden kann, dass sowohl Harnstoffablagerungen als auch eine übermäßige Temperaturbeanspruchung des Förderorgans vermieden wird.

Die Aufgabe wird dadurch gelöst, dass stromwärts vor dem Wäscher ein Teilstrom aus dem Prozessgasstrom abgezweigt wird, am Wäscher vorbei geführt und stromwärts vor dem Wirbelbettreaktor in den zur Verwirbelung dienenden und im Wäscher behandelten Prozessgasstrom eingeführt wird. Ein Vorteil dieses Verfahrens ist es, dass über die Einstellung des Mischungsverhältnisses der Gasströme eine Gastemperatur eingestellt werden kann, durch die Harnstoffablagerungen am Eintritt des Förderorgans vermieden werden und die das Förderorgan keiner zu hohen Temperaturbelastung aussetzt.

Ein weiterer Vorteil dieser Erfindung gegenüber dem BASF-Niederdruckverfahren ist, dass dem am Wäscher vorbei geführten Prozessgas nicht unnötig Wärme entzogen wird, die im Wirbelbettreaktor wieder zugeführt werden müsste. Der Isocyansäureanteil dieses Teilstroms wird nicht im Wäscher in Harnstoff zurück verwandelt, sondern kann direkt im Wirbelbettreaktor in Melamin umgesetzt werden. Ein weiterer Vorteil der Erfindung besteht darin, dass der Harnstoffwäscher, da er mit weniger Prozessgas beaufschlagt wird, kleiner und damit kostengünstiger gebaut werden kann.

Eine vorteilhafte Ausgestaltung der Erfindung ist dadurch gekennzeichnet, dass in der Rohrleitung des am Wäscher vorbei geführten Teilstroms, zu dessen Kühlung, ein indirekter Wärmetauscher installiert ist. Durch dessen Kühlung kann sein Anteil am Verwirbelungsgas gegenüber dem Anteil des im Wäscher behandelten Gases erhöht werden, ohne die Temperatur des Gemisches zu verändern. Auf diese Weise kann eine Feineinstellung des Gemisches zur Optimierung des Prozesses hinsichtlich Energieverbrauch, Produktausbeute-und reinheit und Ablagerungen in den Apparaten erfolgen.

Eine weitere vorteilhafte Ausgestaltung des Verfahrens ist dadurch gekennzeichnet, dass in die Rohrleitung des Teilstroms Ammoniak eingespeist wird. Diese Einspeisung erfolgt alternativ oder ergänzend zur Verwendung eines Wärmetauschers und dient dem gleichen Zweck. Die Einspeisung von Ammoniak, direkt oder zusammen mit dem Harnstoff, in den Wirbelbettreaktor, kann damit verringert werden.

Eine weitere vorteilhafte Ausgestaltung des Verfahrens ist dadurch gekennzeichnet, dass stromwärts vor dem Förderorgan des Verwirbelungsgases, in der Rohrleitung ein statischer Mischer installiert ist, um eine gute Durchmischung des im Wäscher behandelten und des am Wäscher vorbei geführten Gases zu erreichen.

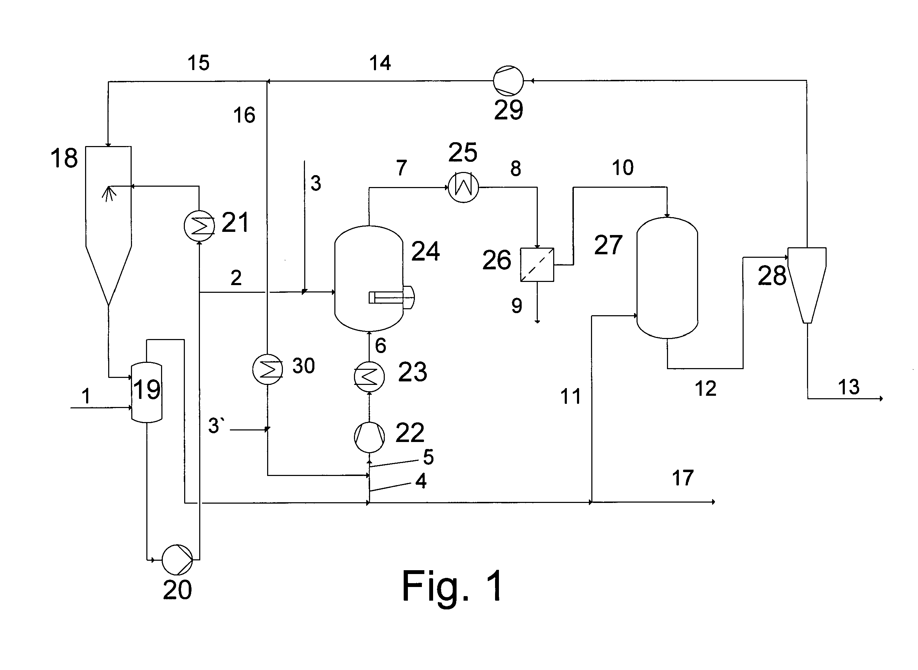

Im Folgenden wird an einem Beispiel mit Hilfe der Zeichnung Fig. 1 und der dazugehörigen Stoffstromtabelle das erfindungsgemäße Verfahren erläutert.

Zeichenerklärung für Fig. 1:
- 18: Wäscher
- 19: Gasabscheider
- 20: Pumpe
- 21: Kühler
- 22: Verdichter oder Gebläse
- 23: Erhitzer
- 24: Wirbelschichtreaktor mit Erhitzer
- 25: Kühler
- 26: Abscheider
- 27: Kristallisator
- 28: Abscheider
- 29: Verdichter oder Gebläse
- 30: Kühler

Flüssiger Harnstoff, als Ausgangsmaterial für die Melaminherstellung, wird in den Gasabscheider (19) eingespeist und wird mittels Pumpe (20) über einen Kühler (21) als Waschflüssigkeit zur Verwendung in Wäscher (18) im Kreis geführt. Im Wäscher (18) werden, durch eine exotherme chemische Reaktion, im Prozessgasstrom (15) vorhandene Reste an Isocyansäure in Harnstoff umgesetzt. Die dabei entstandene Reaktionswärme wird mittels Kühler (21) aus dem im Kreis geführten Harnstoff abgeführt. Aus dem Harnstoffkreislauf des Wäschers wird ein Harnstoffstrom (2) abgezweigt, mit einem Ammoniakstrom (3) versetzt und in den Wirbelbettreaktor (24) gegeben. Aus dem im Wäscher (18) behandelten Prozessgasstrom (4) und dem am Wäscher vorbei geleiteten Prozessgasstrom (16) wird der Prozessgasstrom (5) gebildet, der dem Verdichter oder Gebläse (22) vorgelegt wird. Dabei wird das Mischungsverhältnis der Stoffströme (4) und (16) so eingestellt, dass dem Verdichter oder Gebläse (22) der Prozessgasstrom (5) mit einer Temperatur von 200°C vorgelegt wird. Bei dieser Temperatur besteht keine Gefahr der Bildung von Harnstoffablagerungen oder thermischer Überbeanspruchung des Förderorgans. Der Prozessgasstrom (16) kann mittels Kühler (30) und/oder durch Einspeisung kühlen, gasförmigem oder flüssigen Ammoniak, Stoffstrom (3') gekühlt werden, sodass sein Mengenanteil gegenüber Stoffstrom (4) erhöht werden kann, ohne die Temperatur des Gasgemisches zu verändern.

Das Prozessgas (5) wird über den Erhitzer (23) als Verwirbelungsgas in den Wirbelbettreaktor (24) gefördert. Im Wirbelbettreaktor wird in einem endothermen chemischen Prozess, in Anwesenheit eines Katalysators, Harnstoff, über das Zwischenprodukt Isocyansäure in gasförmiges Melamin und Nebenprodukte, hauptsächlich Melem und Melam, umgesetzt. Der Wirbelbettreaktor ist mit einem Erhitzer ausgestattet, der die notwendige Reaktionswärme liefert. Das mit den Reaktionsprodukten beladene Prozessgas verlässt als Stoffstrom (7) den Reaktor, wird in Kühler (25) soweit abgekühlt, dass die Nebenprodukte auskristallisieren, so dass diese im nachfolgenden Abscheider (26), zusammen mit aus dem Wirbelbettreaktor mitgerissenen Katalysatorpartikeln, abgeschieden werden können. Das danach noch mit Melamin beladene Prozessgas wird in den Kristallisator (27) geleitet, in dem es mit einem, aus dem Wäscher (18) kommendem, Prozessgas vermischt und gekühlt wird. Dabei kommt es zum Auskristallisieren des Melamins, das im nachfolgenden Abscheider (28) aus dem Prozessgas abgetrennt wird und nach Verlassen des Abscheiders als Verfahrensprodukt Stoffstrom (13) vorliegt. Aus dem Abscheider (29) wird das Prozessgas mittels des Verdichters oder Gebläses (29) als Stoffstrom (14) zurück zum Anfang des Prozesses geführt, um als Stoffstrom (15) im Wäscher (18) behandelt zu werden und als Stoffstrom (16) am Wäscher vorbei geführt zu werden. Durch diese Kreislaufführung des Prozessgases, liegt Prozessgas im Überschuss vor, sodass ein Teil, als Stoffstrom (17), aus dem Prozessgaskreislauf ausgeschleust werden muss.

**Stoffstromtabelle**):**

| Stoffstrom -Nr. | | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|---|
| Harnstoff | | x | x | | | | | | | | | | | | | | | |
| Ammoniak | | | | x | | | | | | | | | | | | | | |
| Prozessgas (NH₃ + CO₂) | | | | | x | x | x | x | x | | x | x | x | | x | x | x | x |
| Melamin | | | | | | | | x | x | | x | | x | x | | | | |
| Nebenprodukt + Katalysator-reste | | | | | | | | x | x | x | | | | | | | | |
| Durchfluss | t/h | 10 | 11 | 0,2 | 16 | 31,1 | 31,1 | 37 | 37 | 0,04 | 37 | 105 | 142 | 3,2 | 139 | 123,7 | 15,1 | 7,0 |
| Temp. | °C | 138 | 138 | 150 | 138 | 200 | 390 | 390 | 340 | 340 | 340 | 138 | 210 | 210 | 231 | 231 | 231 | 138 |
| Aggregatzustand | *) | fl | fl | g | g | g | g | g | g, f | f | g | g | g, f | f | g | g | g | g |

| | | | | | | | | | | | | | | | | | | |
|---|---|---|---|---|---|---|---|---|---|---|---|---|---|---|---|---|---|---|
| *) fl = flüssig g = gasförmig f = fest **) Beispielhafte Verfahrensweise, ohne Verwendung des Kühlers (30) und ohne Einspeisung von Ammoniak (3') in Teilstrom (16) | | | | | | | | | | | | | | | | | | |

## Patentansprüche

1. Verfahren zur kontinuierlichen Herstellung von Melamin, wobei der als Ausgangsmaterial verwendete, flüssige Harnstoff in einem Wirbelbettreaktor (24), in Anwesenheit eines Katalysators, unter Wärmezufuhr und unter Zugabe von Ammoniak in ein Prozessgas, bestehend hauptsächlich aus Melamin, Ammoniak, Kohlendioxid, dem Zwischenprodukt Isocyansäure und Nebenprodukten, umgewandelt wird und wobei dieses Prozessgas, nachdem aus ihm die Nebenprodukte und das Melamin abgetrennt wurden, in einen Wäscher (18) geleitet wird, in dem es mit flüssigem Harnstoff gewaschen und vom Isocyansäuregehalt befreit wird, wobei das Prozessgas, nach Verlassen des Wäschers (18), zum Teil als Verwirbelungsgas (4) im Wirbelbettreaktor (24) eingesetzt wird, zum Teil als Kühlgas im Kristallisator (27) eingesetzt wird und zum Teil aus dem Prozessgaskreislauf ausgeschleust wird (17), **dadurch gekennzeichnet, dass** stromwärts vor dem Wäscher (18) ein Teilstrom (16) aus dem Prozessgasstrom (14) abgezweigt wird, am Wäscher (18) vorbei geführt und stromwärts vor dem Wirbelbettreaktor (24) in den zur Verwirbelung dienenden und im Wäscher (18) behandelten Prozessgasstrom (4) eingeführt wird.

2. Verfahren nach Anspruch 1, **dadurch gekennzeichnet, dass** in der Rohrleitung des Teilstroms (16), zu dessen Kühlung, ein indirekter Wärmetauscher (30) installiert ist.

3. Verfahren nach Anspruch 1 oder 2, **dadurch gekennzeichnet, dass** in die Rohrleitung des Teilstroms (16) Ammoniak (3') eingespeist wird.

4. Verfahren nach einem der vorhergehenden Ansprüche, **dadurch gekennzeichnet, dass** stromwärts vor dem Förderorgan (22) in Leitung (5) ein statischer Mischer installiert ist.

## Claims

1. A process for the continuous production of melamine, wherein in a fluidized-bed reactor (24), in the presence of a catalyst, by supplying heat and by adding ammonia, the liquid urea used as starting material is converted into a process gas, chiefly consisting of melamine, ammonia, carbon dioxide, the intermediate product isocyanic acid and by-products, and wherein this process gas, after the by-products and the melamine have been separated therefrom, is passed into a scrubber (18) in which it is washed with liquid urea and liberated from the isocyanic acid content, wherein the process gas, after leaving the scrubber (18), in part is used as fluidizing gas (4) in the fluidized-bed reactor (24), in part is used as cooling gas in the crystallizer (27), and in part is discharged (17) from the process gas circuit, **characterized in that** in flow direction before the scrubber (18) a partial stream (16) is branched off from the process gas stream (14), guided past the scrubber (18), and in flow direction before the fluidized-bed reactor (24) introduced into the process gas stream (4) serving for fluidization and treated in the scrubber (18).

2. The process according to claim 1, **characterized in that** in the conduit of the partial stream (16), for cooling the same, an indirect heat exchanger (30) is installed.

3. The process according to claim 1 or 2, **characterized in that** ammonia (3') is fed into the conduit of the partial stream (16).

4. The process according to any of the preceding claims, **characterized in that** in flow direction before the conveying means (22) a static mixer is installed in conduit (5).

## Revendications

1. Procédé de production en continu de mélamine, dans lequel on transforme de l'urée liquide utilisée comme matière de départ, dans un réacteur (24) à lit fluidisé, en présence d'un catalyseur, avec apport de chaleur et addition d'ammoniac en un gaz de processus constitué principalement de mélamine, d'ammoniac, de dioxyde de carbone, du produit intermédiaire acide isocyanique et de sous-produits, et dans lequel on envoie ce gaz de processus, après en avoir séparé les sous-produits et la mélamine, à un laveur (18) dans lequel on le lave par de l'urée liquide et on le débarrasse de sa teneur en acide isocyanique, le gaz de processus, après avoir quitté le laveur (18), étant utilisé en partie comme gaz (4) de fluidisation dans le réacteur (24) à lit fluidisé, étant utilisé comme gaz de refroidissement dans le cristalliseur (27) et étant évacué (17) en partie du circuit de gaz de processus, **caractérisé en ce qu'**en amont du laveur (18), on dérive un courant (16) partiel du courant (14) de gaz de processus, on le fait contourner le laveur (18) et on l'envoie, en amont du réacteur (24) à lit fluidisé, dans le courant (4) de gaz de processus servant à la fluidisation et traité dans le laveur (18).

2. Procédé suivant la revendication 1, **caractérisé en ce que** l'on installe dans la canalisation du courant (16) partiel, pour son refroidissement, un échangeur de chaleur (30) indirect.

3. Procédé suivant la revendication 1 ou 2, **caractérisé en ce que** l'on injecte de l'ammoniac (3') dans la canalisation du courant (16) partiel.

4. Procédé suivant l'une des revendications précédentes, **caractérisé en ce que** l'on installe un mélangeur statique en amont de l'organe (22) de transport dans le conduit (5).
